(19) Europäisches Patentamt
European Patent Office
Office européen des brevets

(11) Numéro de publication: **0 179 009**
**A1**

(12) # DEMANDE DE BREVET EUROPEEN

(21) Numéro de dépôt: 85450019.6

(22) Date de dépôt: 02.09.85

(51) Int. Cl.⁴: C 07 D 295/08
A 61 K 31/495

(30) Priorité: 19.09.84 FR 8414579
26.07.85 FR 8511574

(43) Date de publication de la demande:
23.04.86 Bulletin 86/17

(84) Etats contractants désignés:
BE CH DE GB IT LI LU NL

(71) Demandeur: SOCIETE CORTIAL S.A.
7 rue de l'Armorique
F-75015 Paris(FR)

(72) Inventeur: Creuzet, Marie-Hélène
Résidence Jardin de Gambetta T3
F-33000 Bordeaux(FR)

(72) Inventeur: Feniou, Claude
5 rue du Général Guillaumat
F-33600 Pessac(FR)

(72) Inventeur: Guichard, Françoise
26 avenue Eugène Delacroix
F-33700 Merignac(FR)

(72) Inventeur: Pontagnier, Henri
21 rue Edouard Vaillant
F-33600 Pessac(FR)

(72) Inventeur: Prat, Gisèle
Hameau de Noailles Villa 18
F-33400 Talence(FR)

(74) Mandataire: Tajan, Marie-Thérèse
LABORATOIRES SARGET Avenue du Président JF Kennedy
F-33701 Mérignac(FR)

(54) **Nouvelles n1-(aminoalcoxy)-4 isopropyl-5 phénoxy)-2 éthyl n4-phényl pipérazines, leur méthode de préparation et leur application thérapeutique.**

(57) La présente invention concerne de nouvelles N1-((aminoalcoxy)-4 isopropyl-5 méthyl-2 phénoxy)-2 éthyl N4-phényl pipérazines, leur méthode de préparation et leur application thérapeuique.

Les produits faisant l'objet de la présente invention ont pour formule

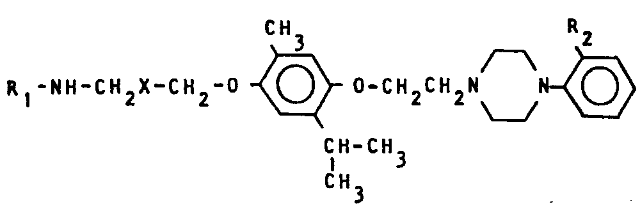

dans laquelle $R_1$ représente un groupment alkyle inférieur ramifié tel qu'isopropyle ou tertiobutyle, $R_2$ représente un groupement alcoxy inférieur choisi de préférence dans le groupe méthoxy, éthoxy, propoxy et X représente $-CHOH-$ ou $-(CH_2)_n-$avec n=0, 1 ou 2.

Ces nouveaux dérivés peuvent être sous forme de base libre ou de sels pharmaceutiquement compatibles tels que leurs chlorhydrates.

Les produits faisant l'objet de la présente invention sont utiles en thérapeutique humaine et vétérinaire.

EP 0 179 009 A1

La présente invention concerne de nouveaux dérivés (alcoxy-2) phénylpipéraziniques, leur méthode de préparation et leur application thérapeutique.

Les nouveaux produits faisant l'objet de la présente invention ont pour formule générale (I)

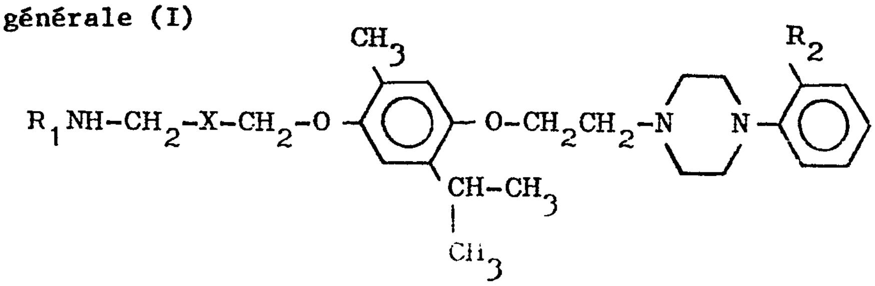

(I)

dans laquelle $R_1$ représente un groupement alkyle inférieur ramifié tel qu'isopropyle ou tertiobutyle, $R_2$ représente un groupement alcoxy inférieur choisi de préférence dans le groupe méthoxy, éthoxy, propoxy et X représente -CHOH- ou $-(CH_2)_n$ avec n = 0, 1 ou 2. Ces produits peuvent être sous forme de base libre ou de sels d'acides pharmaceutiquement compatibles tels que leurs chlorhydrates.

Les nouveaux produits de formule générale I présentent des propriétés antihypertensives de très longue durée liées à leurs propriétés de blocage des récepteurs alpha-1 et béta-adrénergiques.

On connait déjà des produits associant les propriétés alpha et bétabloquantes. Ainsi le labétalol (brevet US 4 012 444) est utilisé en thérapeutique dans le traitement de l'hypertension. D'autre part le brevet FR n° 7902733 déposé par la société Hoffmann-la-Roche le 2 février 1979 concerne en particulier des dérivés de phényl pipérazine de formule générale (II)

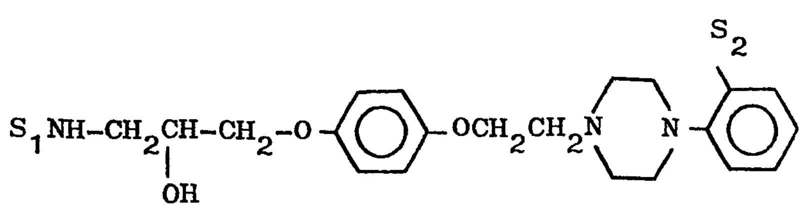

(II)

avec $S_1$ = alkyle inférieur et $S_2$ = hydrogène ou alcoxy inférieur.

Les dérivés faisant l'objet de la présente invention et tels que X représente -CHOH- diffèrent de ceux décrits dans le brevet FR n° 7902733 par la présence de substituants méthyle et isopropyle sur le noyau paraphénylènedioxy, ceci entrainant de façon tout à fait imprévisible des propriétés alpha-1 et bétabloquantes très importantes se manifestant à des doses très faibles.

Les produits faisant l'objet de la présente invention sont préparés de façon générale par réaction entre une ((halogénoéthoxy)-4 isopropyl-5 méthyl-2 phénoxy)alkylamine de formule générale (III)

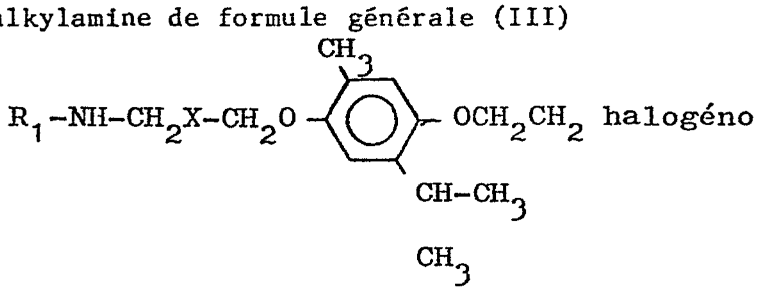

(III)

dans laquelle $R_1$ représente un groupement alkyl inférieur ramifié tel qu'isopropyle ou tertiobutyle, X représente -CHOH- ou $-(CH_2)_n-$ avec n = 0, 1 ou 2 et halogéno représente un atome de chlore ou de brome et une (alcoxy-2 phényl) pipérazine de formule (IV)

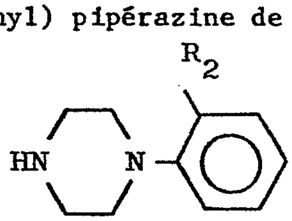

(IV)

dans laquelle $R_2$ représente un groupement alcoxy inférieur choisi de préférence dans le groupe méthoxy, éthoxy, propoxy, en présence d'une base telle que la triéthylamine et d'un solvant tel que l'éthanol.

Ces produits peuvent également être préparés à partir d'un ((alcoxy-2 phényl) pipérazinyl-2 éthoxy)-4 isopropyl-5 méthyl-2 phénol de formule générale (V)

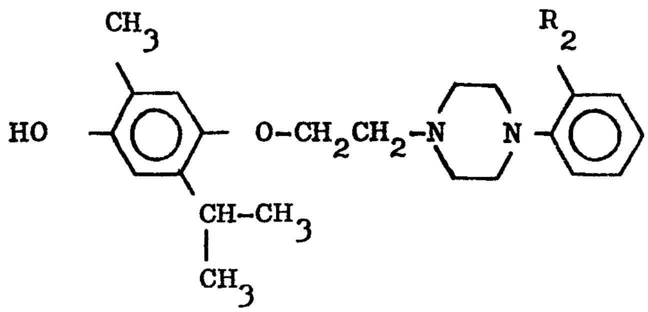

(V)

dans laquelle $R_2$ représente un groupement alcoxy inférieur choisi de préférence dans le groupe méthoxy, éthoxy, propoxy. Dans le cas où X représente $(CH_2)_n$ avec n = 0, 1 ou 2, on fait réagir ce phénol avec un dibromoalcane de formule générale (VI)

$$Br-CH_2-X-CH_2-Br \qquad (VI)$$

puis l'on fait réagir le dérivé bromé ainsi obtenu avec une alkylamine de formule générale VII

$$R_1NH_2 \qquad (VII)$$

dans laquelle $R_1$ représente un groupement alkyl inférieur ramifié tel qu'isopropyle ou tertiobutyle.

Dans le cas où X représente -CHOH-, on peut préparer à partir du phénol de formule générale (V), l'époxyde de formule générale (VIII)

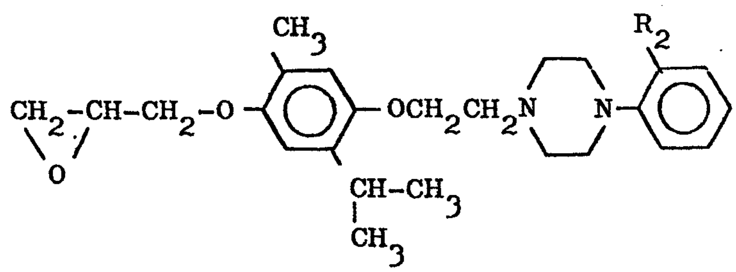

(VIII)

Cet époxyde réagit ensuite avec l'alkylamine de formule générale (VII) pour donner le produit de formule générale (I) tel que X = CHOH. L'époxyde (VIII) peut être obtenu par plusieurs méthodes. On peut par exemple faire réagir un phénol de formule générale (V) avec du bromure d'allyle en présence d'acétone et de carbonate de potassium de façon à préparer son éther d'allyle ; celui-ci est ensuite transformé en époxyde par réaction avec du N-bromoacétamide et de

l'acide perchlorique. On peut également selon une autre variante faire réagir un phénol de formule générale (V) avec l'épichlorhydrine

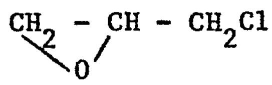

en présence de NaH dans un solvant tel que le diméthylformamide.

Les exemples ci-après vont permettre de mieux illustrer la présente invention sans toutefois en restreindre la portée.

Exemple 1

Préparation du dichlorhydrate de l'(isopropyl-5 (((méthoxy-2 phényl) pipérazinyl)-2 éthoxy)-4 méthyl-2 phénoxy)-1 isopropylamino-3 propanol-2 ; produit de formule I avec $R_1$ = isopropyle, $R_2$ = méthoxy, X = CHOH, sous forme de dichlorhydrate. Nom de code COR28 48.

Synthèse

On chauffe pendant 11 heures au reflux le mélange formé de 2,7 g de (bromo-2 éthoxy)-4 isopropyl-5 méthyl-2 phénol, 20 ml d'acétone, 2,5 ml de bromure d'allyle et 2,2 g de carbonate de potassium. Le solvant est éliminé par évaporation sous vide de la trompe à eau puis le résidu est repris dans 100 ml d'eau.

La phase aqueuse obtenue est extraite deux fois par 50 ml d'éther éthylique. Les phases organiques réunies sont lavées par de la soude 1N , par de l'eau jusqu'à neutralité, sont séchées sur sulfate de sodium puis distillées.

On obtient avec un rendement de 96% un résidu consistant en l'éther d'allyle du (bromo-2 éthoxy)-4 isopropyl-5 méthyl-2 phénol suffisamment pur pour être utilisé pour la suite de la synthèse.

7,5 g de N-bromoacétamide sont ajoutés en 12 mn au mélange constitué par 11,2 g de l'éther d'allyle précédemment décrit, 180 ml d'acétone et 0,36 ml d'acide perchlorique à 70%.

Ce milieu réactionnel est maintenu sous agitation à température ambiante pendant 2,5 heures puis l'acide perchlorique est neutralisé par du bisulfite de sodium. A ce moment le solvant est éliminé par distillation sous vide.

Le résidu obtenu est repris dans 100 ml d'eau et cette phase aqueuse est extraite deux fois par 50 ml d'éther éthylique.

Les phases éthérées sont réunies, séchées sur sulfate de sodium puis distillées. Le résidu huileux est dissous dans 250 ml de méthanol auxquels 73 ml de soude 1N sont ajoutés. Cette solution est maintenue sous agitation à température ambiante pendant 2 heures puis le méthanol est distillé.

Le nouveau résidu de distillation est dissous dans 200 ml d'eau. La phase aqueuse est extraite par deux fois 50 ml d'éther éthylique. Les phases organiques réunies sont lavées à l'eau, séchées pui distillées. Le produit résiduel qui consiste en l'époxyde attendu est purifié en chromatographie sur colonne ouverte de silice par élutions successives par du toluène pur, puis par un gradient d'acétate d'éthyle dans le toluène.

Le rendement global de cette étape est de 46% par rapport à l'éther d'allyle mis en jeu.

Le mélange formé de 6,7 g de l'époxyde ci dessus décrit, 1,8 g d'isopropylamine et 50 ml de méthanol est maintenu sous agitation sous argon pendant 3 heures à 60°C.

Après distillation sous vide le résidu est repris dans l'acide chlorhydrique dilué, nouvelle phase qui est extraite successivement par deux fois 100 ml d'éther éthylique puis par deux fois 100 ml de chloroforme. Les phases éthérées sont écartées. Les phases chloroformiques sont réunies, séchées puis concentrées. A cet instant de l'éther éthylique est ajouté à chaud au milieu réactionnel jusqu'à apparition d'un léger trouble. Le milieu réactionnel est refroidi à 5°C.

Le précipité blanc formé est alors filtré, dissous dans 50 ml d'eau qui est ensuite alcalinisée par addition de soude 1N. Cette solution aqueuse est extraite par deux fois 30 ml d'éther éthylique. Les phases éthérées sont réunies, lavées par l'eau jusqu'à neutralité, séchées puis distillées.
On obtient avec un rendement de 47% le ((bromo-2 éthoxy)-4 isopropyl-5 méthyl-2 phénoxy)-1 isopropylamino-3 propanol-2.

Le mélange constitué par 3,88 g de ((bromoéthoxy)-4 isopropyl-5 méthyl-2 phénoxy)-1 isopropylamino-3 propanol-2, 1,92 g de (méthoxy-2 phényl) pipérazine, 10 ml de triéthylamine et 20 ml d'éthanol est maintenu au reflux pendant 4 heures. La réaction est suivie par chromatographie sur couches minces de silice (éluant : $CHCl_3$ = 80/$CH_3OH$ = 20/Pic B7) et en chromatographie liquide haute performance sur colonne G8 (éluant : $CH_3OH$ =70/$H_2O$ = 30, V/V, débit = 1,5 ml/mn).

Le mélange est refroidi à température ambiante puis alcalinisé. Le solvant est distillé puis le résidu est repris dans 100 ml de chloroforme. La phase chloroformique est lavée à l'eau jusqu'à neutralité, séchée et concentrée. L'addition d'éther éthylique ainsi que le refroidissement du milieu réactionnel à 5°C entraînent la formation d'un précipité qui est filtré lavé à l'éther éthylique et séché.

On obtient avec un rendement de 52 % par rapport au dérivé bromé mis en jeu l'(isopropyl-5 (((méthoxy-2 phényl) pipérazinyl)-2 éthoxy)-4 méthyl-2 phénoxy)-1 isopropylamino-3 propanol-2 qui est transformé en son dichlorhydrate par passage d'un courant d'HCl gazeux sur sa solution méthanolique.

Propriétés physicochimiques

Point de fusion du dichlorhydrate mesuré sur appareil Mettler : 88°C

Spectre de RMN du dichlorhydrate dans le $DMSOD_6$ : 1,2 et 1,3 ppm, 12 protons, doublets, 2 ($C(CH_3)_2$) ; 2,2 ppm, 3 protons, singulet, $CH_3$ phénylique ; 2,7 -4,7 ppm, 22 protons, massif complexe, 8 $CH_2$ + 3 CH + $OCH_3$ sortant à 3,8 ppm ; 6,5 ppm, 1 proton, pic large, OH, échangeable avec $D_2O$ ; 6,7 -7,2 ppm, 6 protons, massif complexe, protons aromatiques ; 9,2 ppm, 2 protons, dôme, $^+NH_2$, échangeable avec $D_2O$ ; 12 ppm, 1 proton, dôme, $^+NH$, échangeable avec $D_2O$.

Exemple 2

Dichlorhydrate de l'((((éthoxy-2 phényl) pipérazinyl)-2 éthoxy)-4 isopropyl-5 méthyl-2 phénoxy)-1 isopropylamino-3 propanol-2 ou COR28 75 ; dichlorhydrate du produit de formule (I) dans laquelle $R_1$ = isopropyl, $R_2$ = éthoxy, X = CHOH.

Synthèse

Le mélange constitué par 16 g de ((bromo-2 éthoxy)-4 isopropyl-5 méthyl-2 phénoxy)-1 isopropylamino-3 propanol-2 préparé selon l'exemple 1, 8,5 g d'(éthoxy-2 phényl) pipérazine, 100 $cm^3$ d'éthanol et 5 $cm^3$ de triéthylamine est chauffé pendant 6 heures au reflux. Le solvant est évaporé. Le résidu est dissous dans de l'acide chlorhydrique dilué (1N), puis est extrait deux fois à l'éther puis deux fois au chloroforme. Le chloroforme est évaporé ; le chlorhydrate est neutralisé et la base est purifiée sur colonne de silice en éluant par le mélange chloroforme (90) - méthanol (10). On obtient ainsi 4 g d'((((éthoxy-2 phényl) pipérazinyl)-2 éthoxy)-4 isopropyl-5 méthyl-2 phénoxy)-1 isopropylamino-3 propanol-2. Rendement 19 %. Le chlorhydrate est préparé en dissolvant la base dans de l'éther et en faisant passer un courant d'acide chlorhydrique gazeux jusqu'à précipitation complète. Il est ensuite filtré et lavé à l'éther.

Propriétés physicochimiques

Produit hygroscopique

Spectre de RMN du dichlorhydrate dans le $DMSOD_6$: 1,0 - 1,7 ppm, 15 protons, massif complexe, 2 $C(CH_3)_2$ + $CH_3$ du groupement éthoxy -2 phényl ; 2,2 ppm, 3 protons, singulet, $CH_3$ sur le cycle thymol ; 2,9-4,8 ppm, 21 protons, massif complexe, 9 $CH_2$ + -3CH- ; 6,7 - 7,2 ppm, 6 protons, massif complexe, protons aromatiques ; 9,3 ppm, 2 protons, dôme, $^{+}NH_2$, échangeable avec $D_2O$ ; 12,2 ppm, 1 proton, dôme, $^{+}NH$, échangeable avec $D_2O$.

Exemple 3

Dichlorhydrate de la N-isopropyl N-(((((méthoxy-2 phényl) pipérazinyl)-2 éthoxy)-4 isopropyl-5 méthyl-2 phénoxy)-3 propyl) amine ou COR28 79 ; dichlorhydrate du produit de formule (I) avec $R_1$ = isopropyl, $R_2$ = méthoxy, X = $CH_2$.

Synthèse

Dans un ballon tricol, on ajoute 12 g de ((méthoxy-2 phényl) pipérazinyl)-2 éthoxy)-4 isopropyl-5 méthyl-2 phénol et 31 g de dibromo-1,3 propane. On chauffe à 140 °C, puis on rajoute goutte à goutte une solution de

2,5 g de potasse dissoute dans 26 $cm^3$ de méthanol. La réaction est assez vive. On maintient le mélange réactionnel sous agitation pendant 1 h au reflux, puis on le filtre à chaud. Le précipité est rincé avec 10 $cm^3$ de méthanol puis avec 5 $cm^3$ de dibromo-1,3 propane. Le filtrat est porté au reflux sous agitation pendant 10 h. Il est refroidi. On lui additionne 50 ml de chloroforme et 50 ml d'eau. La phase chloroformique est décantée et lavée à plusieurs reprises avec 30 ml de soude glacée à 5 %. La phase chloroformique est ensuite lavée à l'eau, séchée ; le chloroforme est évaporé. L'excès de dibromopropane est distillé à la pompe à palettes. Le produit brut est purifié sur colonne de silice avec pour éluant du chloroforme. Rendement 30 %.

On mélange dans un réacteur 6 g de dérivé bromopropoxy ainsi préparé, 30 $cm^3$ d'éthanol, 20 $cm^3$ d'isopropylamine et 6 $cm^3$ de triéthylamine. Le mélange est porté au reflux sous agitation pendant 10 h, puis laissé refroidir. L'alcool est évaporé et le résidu est repris par de l'éther ; il est extrait par une solution aqueuse d'acide chlorhydrique. La phase acide est extraite au chloroforme. La phase chloroformique est séchée, concentrée, puis additionnée d'éther. Le chlorhydrate précipite. Il est filtré, rincé à l'éther et séché. Rendement 60 %.

Propriétés physicochimiques

Point de fusion du dichlorhydrate mesuré en utilisant un appareil Mettler FP5 : 107,8 - 108,2 °C.

Spectre de RMN du dichlorhydrate dans le $DMSOD_6$: 1,2 ppm, 6 protons, doublet, $C(CH_3)_2$ sur un cycle aromatique ; 1,3 ppm, 6 protons, doublet, $NC(CH_3)_2$ ; 1,8 - 2,5 ppm, 5 protons, massif complexe, $CH_3$ sur un cycle phényle et $CCH_2C$ ; 2,8-4,7 ppm, 21 protons, massif complexe, 6 $CH_2$-N + 2 -CH- + 2 $OCH_2$ + $OCH_3$ sortant à 3,9 ppm ; 6,8 - 7,3 ppm, 6 protons, massif complexe, protons aromatiques ; 9,1 ppm, 2 protons, dômes, $NH^+$ et $^+NH_2$, échangeables avec $D_2O$.

Exemple 4

Variante pour synthétiser le produit de l'exemple 2 ou COR 2875

A une solution de 210 ml de diméthylformamide anhydre contenant 32 g d'(((éthoxy-2 phényl)pipérazinyl)-2 éthoxy)-4 isopropyl-5 méthyl-2 phénol, on ajoute peu à peu et à température ambiante, 298,5 g de NaH. On agite pendant environ 15 mn à température ambiante. On ajoute 21 ml d'épichlorhydrine et on chauffe pendant 1 heure à 80 °C. Le solvant est évaporé à la pompe à palettes. Le résidu est dissous dans 650 ml d'éther, puis lavé à l'eau. Les phases éthérées sont séchées. Le solvant est évaporé. On obtient ainsi l'époxyde avec un rendement de 96 %.

Le mélange constitué par 35 g d'époxyde ainsi préparé, 41 ml d'isopropylamine et 200 ml de méthanol est chauffé au reflux sous agitation pendant une heure. Le solvant est évaporé. On ajoute 800 ml d'eau et de l'acide chlorhydrique concentré jusqu'à pH acide. On extrait deux à trois fois par 100 ml de chloroforme ; la phase aqueuse est neutralisée avec de l'ammoniaque puis on extrait à nouveau deux fois par le chloroforme. Les phases chloroformiques sont réunies, séchées, évaporées ; le résidu est dissous dans 400 ml de méthanol. On fait passer un courant d'acide chlorhydrique gazeux dans la solution ; celle-ci est concentrée au Rotavapor. On ajoute de l'éther. On filtre le précipité et on le sèche. Le COR 2875 est ainsi obtenu avec un rendement de 90 % sous forme d'une poudre hygroscopique.

Les propriétés toxicopharmacologiques des produits faisant l'objet de la présente invention sont décrites ci-après.

<u>Toxicité</u> : Administré à la souris par voie orale à la dose de 100 mg/kg, le COR 2848 n'entraine aucune mortalité. Il entraine 100% de mortalité quand il est administré à 300 mg/kg. Sa DL50 est égale à 216 (185-253) mg/kg. Les DL50 des COR 2875 et COR 2879 sont égales respectivement à 563,2 (486-652) et 89,97 (72,90-111,03) mg/kg pour la voie orale

Administré à la souris par voie intrapéritonéale, le COR 2848 utilisé à la dose de 50 mg/kg n'entraine aucune mortalité. Il entraine 100 % de mortalité à 100 mg/kg.

Par voie intraveineuse chez la souris les DL50 des COR28 48, COR28 75 et COR28 79 sont égales respectivement à 20 (19-21), 26,7 (24-30) et 7,84 (7,11-8,66) mg/kg.

Activité alpha-1 bloquante : In vivo l'activité alpha bloquante est appréciée par la détermination de l'antagonisme de l'hypertension induite par la phényléphrine. Le produit à tester est administré par voie intraveineuse à des rats amyélés et bivagotomisés selon la technique de J.S.Gillespie et T.S. Muir ( Br.J. Pharmac. Chemother., 1967, 30, 78-87). On indique ci-après l' activité maximale et le temps au bout duquel l'activité résiduelle est égale à 50% de l'activité maximale : $3.10^{-7}$ M/kg, -76,1%, 20 mn ; $10^{-6}$ M/kg, -92,7%, 45 mn ; $3.10^{-6}$ M/kg, -112,5%, 110 mn. La DE50 déterminée dans ces conditions pour le COR2848 est de $4,71.10^{-8}$ mole/kg au bout de 2 mn, $7,55.10^{-7}$ mole/kg au bout de 30 mn et $1,57.10^{-6}$ mole/kg au bout de 60 mn.

Activité betabloquante : administré par voie orale à la dose de 25 mg/kg par voie orale, le COR2848 entraine 30 mn après son administration 83 % d'inhibition de la tachycardie induite chez la souris par l'isoprotérénol.

Activité antihypertensive : le produit à tester est administré par voie orale à des rats spontanément hypertendus. La pression artérielle est mesurée de façon indirecte en utilisant un sphygmomanomètre. Pour le COR2848 administré à la dose de 50 mg/kg/j pendant 2 jours, la chute de pression maximale observée est de respectivement 38,5 et 36,7% le premier et le deuxième jour de traitement, 6 heures après le gavage. La pression est significativement abaissée par rapport au lot témoin pendant les deux jours de traitement et de récupération post traitement.

Pour le COR28 79 administré à la dose de 100 mg/kg/j pendant 2 jours, la chute de pression maximale observée est de respectivement 31 et 34 % le premier et le deuxième jour de traitement, 1 heure après le gavage. La pression est significativement abaissée par rapport au lot témoin pendant les deux jours de traitement ; cet abaissement est de 31 % et 24 % respectivement 24 h après le premier et le deuxième gavage. La chute de pression varie entre 11 et 22 % pendant les deux jours de récupération post-traitement.

Activité spasmolytique : in vitro le COR2848 inhibe à la concentration de 2 microg/ml les contractions d'un iléon de cobaye induites par stimulation transmurale électrique dans une solution de Krebs oxygénée.

<u>Activité anticholinergique</u> : in vitro, le COR2848 employé à la concentration de 10 microg/ml inhibe les contractions de segments isolés d'iléons de cobaye induites par 0,1 microg/ml d'acétylcholine.

Compte-tenu de leurs activités pharmacotoxicologiques les produits faisant l'objet de la présente invention sont utiles , seuls ou associés à d'autres principes actifs, par exemple dans le traitement de l'hypertension artérielle ou des arythmies cardiaques ou dans le traitement du glaucome.

Les doses et schémas thérapeutiques seront fonction du sujet et de l'affection à traiter. Les produits pourront être administrés par voie orale (par exemple sous forme de gélules, comprimés, gouttes buvables), par voie injectable (soluté injectable pour la voie intramusculaire ou la voie intraveineuse ; soluté pour perfusion intraveineuse), par voie rectale (suppositoires), par voie locale (collyres, pommades, gels). Suivant les indications, la dose quotidienne variera de 1 à 100 mg en 1 à 3 prises pour la voie rectale ; la dose administrée par voie intraveineuse pourra varier entre 0,1 et 10 mg. Les collyres contiendront 0,05 à 0,5 % de principe actif et les pommades ou gels contiendront 0,5 à 5 % de principe actif.

## REVENDICATIONS

1. Nouveaux produits de formule générale

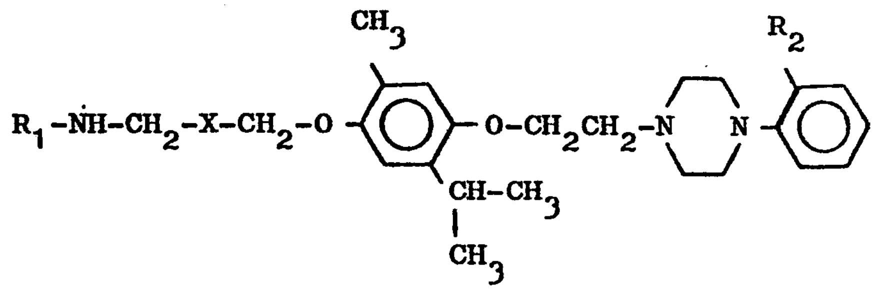

dans laquelle $R_1$ représente un groupement alkyle inférieur ramifié tel qu'isopropyle ou tertiobutyle, $R_2$ représente un groupement alcoxy inférieur choisi de préférence dans le groupe méthoxy, éthoxy, propoxy et X représente -CHOH- ou $-(CH_2)_n-$ avec n = 0, 1 ou 2. Ces produits peuvent être sous forme de base libre ou de sels d'acides pharmaceutiquement compatibles tels que leurs chlorhydrates.

2. Méthode de préparation des produits selon la revendication 1 caractérisée en ce que l'on fait réagir une ((halogénoéthoxy)-4 isopropyl-5 méthyl-2 phénoxy)alkylamine de formule générale

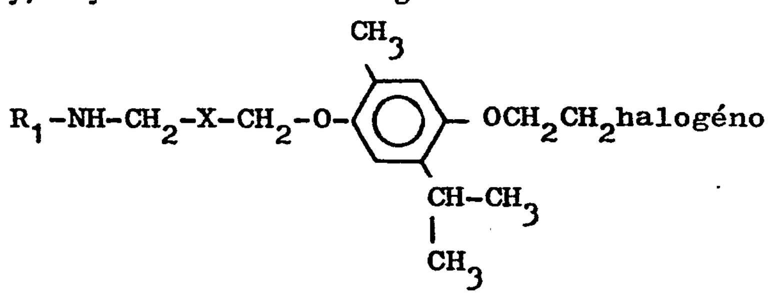

dans laquelle $R_1$ représente un groupement alkyl inférieur ramifié tel qu'isopropyle ou tertiobutyle, X représente -CHOH- ou $-(CH_2)_n-$ avec n = 0, 1 ou 2 et halogéno représente un atome de chlore ou de brome et une (alcoxy-2 phényl) pipérazine de formule

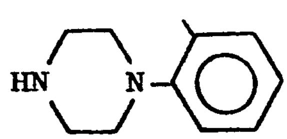

dans laquelle $R_2$ représente un groupement alcoxy inférieur choisi de préférence dans le groupe méthoxy, éthoxy, propoxy, en présence d'une base telle que la triéthylamine et d'un solvant tel que l'éthanol.

3. Méthode de préparation des produits selon la revendication 1 tels que X représente $-(CH_2)_n-$ avec n = 0, 1 ou 2 caractérisée en ce que l'on fait réagir un ((alcoxy-2 phényl) pipérazinyl-2 éthoxy)-4 isopropyl-5 méthyl-2 phénol de formule générale

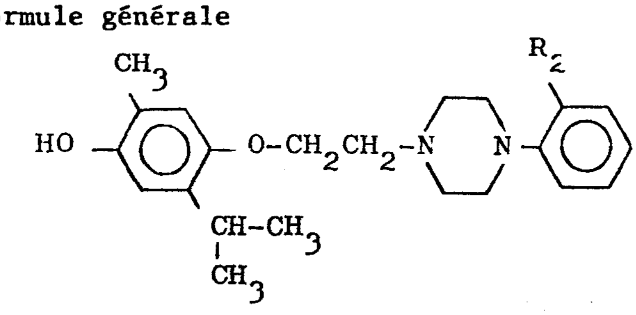

dans laquelle $R_2$ représente un groupement alcoxy inférieur choisi de préférence dans le groupe méthoxy, éthoxy, propoxy avec un dibromoalcane de formule générale

$$Br-CH_2-X-CH_2-Br$$

puis en ce que l'on fait réagir le dérivé bromé ainsi obtenu avec une alkylamine de formule générale

$$R_1NH_2$$

dans laquelle $R_1$ représente un groupement alkyl inférieur ramifié tel qu'isopropyle ou tertiobutyle

4. Méthode de préparation des produits selon la revendication 1 tels que X représente -CHOH- caractérisée en ce que l'on fait réagir un ((alcoxy-2 phényl) pipérazinyl-2 éthoxy)-4 isopropyl-5 méthyl-2 phénol de formule générale

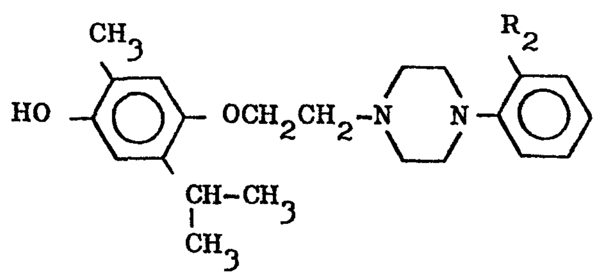

dans laquelle $R_2$ représente un groupement alcoxy inférieur choisi de préférence dans le groupe méthoxy, éthoxy, propoxy avec l'épichlorhydrine

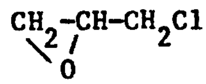

en présence de NaH dans un solvant tel que le diméthylformamide puis en ce que l'on fait réagir l'époxyde ainsi obtenu avec une alkylamine de formule générale

$$R_1NH_2$$

dans laquelle $R_1$ représente un groupement alkyl inférieur ramifié tel qu'isopropyle ou tertiobutyle.

5. Méthode de préparation des produits selon la revendication 1 tels que X représente -CHOH- caractérisée en ce que l'on fait réagir un ((alcoxy-2 phényl) pipérazinyl-2 éthoxy)-4 isopropyl-5 méthyl-2 phénol de formule générale

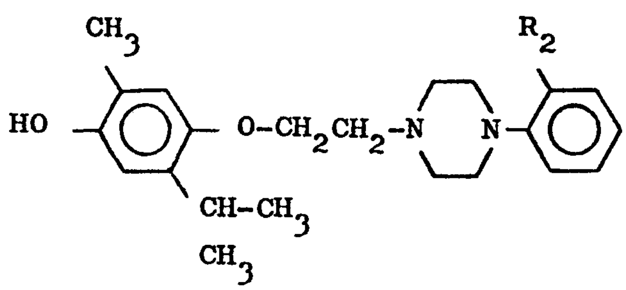

dans laquelle $R_2$ représente un groupement alcoxy inférieur choisi de préférence dans le groupe méthoxy, éthoxy, propoxy avec le bromure d'allyle en présence d'acétone et de carbonate de potassium de façon à préparer l'éther d'allyle du phénol, puis en ce que celui-ci est transformé en époxyde par réaction avec du N-bromoacétamide et de l'acide perchlorique, et enfin en ce que l'on fait réagir l'époxyde ainsi obtenu avec une alkylamine de formule générale

$$R_1NH_2$$

dans laquelle $R_1$ représente un groupement alkyl inférieur ramifié tel qu'isopropyle ou tertiobutyle.

6. Nouveaux médicaments utiles en thérapeutique humaine ou vétérinaire contenant une quantité thérapeutiquement efficace d'au moins un composé selon la revendication 1.

7. Nouveaux médicaments utiles en thérapeutique cardiovasculaire contenant une quantité thérapeutiquement efficace d'au moins un composé selon la revendication 1.

8. Composition pharmaceutique ou vétérinaire caractérisée en ce qu'elle contient à titre de principe actif au moins un produit selon la revendication 1 en association avec un véhicule pharmaceutique ou un excipient approprié.

Office européen des brevets

**RAPPORT DE RECHERCHE EUROPEENNE**

0179009

Numéro de la demande

EP 85 45 0019

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | EP-A-0 062 596 (CORTIAL)<br>* Revendications *<br>--- | 1,6-8 | C 07 D 295/08<br>A 61 K 31/495 |
| Y | CHEMICAL ABSTRACTS, vol. 86, no. 5, 31 janvier 1977, page 334, no. 29467u, Columbus, Ohio, US; & ES - A - 421 546 (ESPECIALIDADES LATINAS MEDI-CAMENTOS UNIVERSALES SA) 01-04-1976<br>--- | 1,6-8 | |
| D,Y | EP-A-0 005 142 (HOFFMANN-LA ROCHE)<br>* Revendications *<br>----- | 1,3-8 | |
| | | | **DOMAINES TECHNIQUES RECHERCHES (Int. Cl.4)** |
| | | | C 07 D 295/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 19-12-1985 | MOREAU J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82